# EUROPÄISCHE PATENTANMELDUNG

(11) **EP 2 528 064 A1**
(43) Veröffentlichungstag der Anmeldung: **28.11.2012**
(21) Anmeldenummer: 12003232.1
(22) Anmeldetag: 03.05.2012
(51) Int. Cl.: G21K 1/10, A61N 5/10

(54) **Multiple Reichweitenmodulatoren**

(30) Priorität: 23.05.2011 DE 102011102977
(71) Anmelder: GSI Helmholtzzentrum für Schwerionenforschung GmbH, 64291 Darmstadt (DE)
(72) Erfinder: Bert, Christoph, 63741 Aschaffenburg (DE); Rietzel, Eike, 64331 (DE); Saito, Nami, 64291 Darmstadt (DE)
(74) Vertreter: Mergel, Volker

(57) **Zusammenfassung**

Die Erfindung gibt eine Vorrichtung und ein Verfahren zum Anpassen der Reichweite eines Partikelstrahls (1) einer Partikelstrahltherapieanlage an. Die Partikelstrahltherapieanlage umfasst mindestens zwei hintereinander angeordnete Reichweitenanpassmittel (11, 12), in denen der Partikelstrahl (1) beim Durchqueren einen Energieverlust erfährt. Vorteilhaft daran ist, dass die Reichweite eines Partikelstrahls (1) dadurch schneller und präziser angepasst werden kann.

## Beschreibung

Die Erfindung betrifft eine im Patentanspruch 1 angegebene Vorrichtung und ein im Patentanspruch 6 angegebenes Verfahren zum Anpassen der Reichweite eines Partikelstrahls einer Partikelstrahltherapieanlage.

Allgemein wird als Strahlentherapie ein medizinisches Therapieverfahren zur Behandlung von Tumorerkrankungen bezeichnet. Dabei wird hochenergetische Photonenstrahlung (Röntgenstrahlung, Gammastrahlung) oder Partikelstrahlung (Elektronen, Protonen, Ionen) auf einen zu behandelnden Körperbereich eines Patienten gelenkt. Partikelstrahltherapieanlagen werden bevorzugt zur Behandlung von Tumoren eingesetzt. Sie besitzen den Vorteil, dass bei Bestrahlung eines Zielobjekts (Target) der größte Teil der Energie des Partikelstrahls auf das Target übertragen wird, während lediglich eine geringe Energie auf gesundes Gewebe übertragen wird. Demzufolge kann eine relativ hohe Bestrahlungsdosis zur Behandlung eines Patienten eingesetzt werden.

Für die Partikelstrahltherapie wird hochenergetische Ionenstrahlung mit einer Beschleunigungsanlage erzeugt. Die auf hohe Energien beschleunigten Partikel werden zu einem Partikelstrahl geformt und anschließend auf das zu bestrahlende Gewebe gerichtet. Die Partikel dringen in das zu bestrahlende Gewebe ein und geben dort in einem umschriebenen Bereich ihre Energie ab. Die Eindringtiefe des Partikelstrahls hängt vornehmlich von der Energie des Partikelstrahls ab. Je höher die Energie des Partikelstrahls, desto tiefer dringen die Partikel in das zu bestrahlende Gewebe ein. Im Vergleich zu herkömmlichen Bestrahlungsverfahren, die mit Röntgen- und Elektronenstrahlen arbeiten, zeichnet sich die Partikelstrahltherapie dadurch aus, dass die Energie der Partikel in einem umschriebenen und abgrenzbaren Bereich abgegeben wird. Hierdurch kann eine Bestrahlung z.B. eines Tumors genauer erfolgen und umliegendes Gewebe kann besser geschont werden.

Um einzelne Volumenelemente eines Zielvolumens oder Tumors gezielt ansteuern zu können und eine Strahlendosis für das Volumenelement optimal anpassen zu können, wurde eine Rasterscannvorrichtung für Partikelstrahlen entwickelt. Mit dieser Vorrichtung wird das Zielvolumen in Schichten gleicher Teilchenreichweite zerlegt und ein feiner, intensitätsgesteuerter Bleistiftstrahl aus Partikeln rasterförmig über die einzelnen Schichten geführt. Zusammen mit der aktiven Energievariation durch einen Partikelbeschleuniger kann damit ein exaktes Ausleuchten in drei Dimensionen eines jeden beliebigen Zielvolumens erreicht werden. Eine präzise 3D-Dosisapplikation ist aber bei einer konstant vorgegebenen kinetischen Energie des Partikelstrahls nur dann gegeben, wenn sich die Lage des Zielvolumens zeitlich nicht ändert. Im Bereich des Kopfes kann dies durch Immobilisation mittels individuell gefertigter Kopfmasken erreicht werden. Bei inneren Organen, die sich beispielsweise infolge der Atmung bewegen, wie zum Beisel die Lunge oder Organe im Thoraxbereich, ist dies jedoch nicht möglich. Beispielsweise ist im Brustbereich eine Bewegung des Zielvolumens besonders problematisch, da das Zielvolumen in den Schattenbereich einer Rippe bewegt werden kann.

Aus dem Stand der Technik sind Vorrichtungen zur Anpassung der Eindringtiefe der Reichweite des Partikelstrahls, d.h. der Position des Bragg-Maximums im bestrahlten Gewebe, vorzugsweise bei bewegten Zielvolumen bekannt. Dabei bezeichnet das Bragg-Maximum den maximalen Energieverlust und somit auch die maximale Schädigung. Während in Strahlrichtung gesehen seitliche Verschiebungen des Zielvolumens mittels einer schnellen Ansteuerung von Rasterscann-Magneten kompensiert werden können, erfordern Verschiebungen in Strahlrichtung eine schnelle Anpassung der spezifischen Energie der Partikel und damit Position des Bragg-Maximums in der Tiefe des Gewebes.

Dies wird mithilfe einer sogenannten Reichweitenmodulation erreicht. Ein feiner Partikelstrahl hoher Energie, wie er etwas von einem Synchrotron oder einem Zyklontronbeschleuniger geliefert wird, erfährt beim Durchgang durch ein Stück homogener Materie einen wohl definierten Energieverlust. Durch Variation der Dicke dieses passiven Reichweitenmodulators, der auch als "Range shifter" bezeichnet wird, lässt sich die Ausgangsgeschwindigkeit der Partikel und damit deren Reichweite im Gewebe einstellen. Die Variation der Dicke wird durch eine keilförmige, stufenförmige oder gekrümmte Struktur des Reichweitenmodulators erreicht.

Eine solche Lösung ist bereits aus der Schrift DE 199 07 098 A1 bekannt. Bei der dort beschriebenen Anordnung besteht der Reichweitenmodulator aus einem Doppelkeilsystem, bei dem zwei auf je einen Linearmotor installierte Plexiglaskeilanordnungen (mit mehreren Keilen) die Strahlenergie und -reichweite modulieren, indem ihre relative Position zueinander und damit die Materialdicke im Überlappbereich der Keile geändert wird.

Bei der Erfassung der lateralen Verschiebung des Zielvolumens ist ein größerer Überlappbereich (ca. 20 x 20 cm²) der Keile erforderlich als bei der longitudinalen Verschiebung des Zielvolumens. Dies kann durch den Einsatz von größer dimensionierten Doppelkeilsystemen erreicht werden. Das Gewicht der Doppelkeilsysteme kann bei solchen Größen die Modulier- und damit die maximale Scanngeschwindigkeit limitieren.

Es ist Aufgabe der Erfindung diese Nachteile zu überwinden und eine verbesserte Vorrichtung zum Anpassen der Reichweite eines Partikelstrahls einer Partikelstrahltherapieanlage anzugeben.

Gemäß der Erfindung wird die gestellte Aufgabe mit der Vorrichtung zum Anpassen der Reichweite eines Partikelstrahls einer Partikelstrahltherapieanlage des unabhängigen Patentanspruchs 1, der Partikelstrahltherapieanlage des unabhängigen Patentanspruchs 5 und dem Verfahren zum Anpassen der Reichweite eines Partikelstrahls einer Partikelstrahltherapieanlage des unabhängigen Patentanspruchs 6 gelöst.

Die Erfindung beansprucht eine Vorrichtung zum Anpassen der Reichweite eines Partikelstrahls einer Partikelstrahltherapieanlage. Die Partikelstrahltherapieanlage umfasst mindestens zwei hintereinander angeordnete Reichweitenanpassmittel, in denen der Partikelstrahl beim Durchqueren einen Energieverlust erfährt. Vorteilhaft daran ist, dass die Reichweite eines Partikelstrahls dadurch schneller und präziser angepasst werden kann.

Vorzugsweise können in der Partikelstrahltherapieanlage die Reichweitenanpassmittel nach einem letzten Ablenkmagneten angeordnet werden.

In einer weiteren vorteilhaften Ausgestaltung der Erfindung können die Reichweitenanpassmittel derart modulierbar sein, dass der Energieverlust variabel einstellbar ist. Dies ermöglicht eine planbare Deposition der Partikelstrahlenergie bei bewegten Zielvolumina.

Vorzugsweise können die Reichweitenanpassmittel unterschiedliche keilförmige Absorptionselemente aufweisen, die ineinander verfahrbar sind. Dadurch ist es möglich, die Partikelstrahlenergie und -reichweite zu verändern, indem die relative Position der keilförmigen Absorptionselemente und damit die Materialdicke im Überlappbereich geändert werden.

Die Erfindung gibt auch eine Partikelstrahltherapieanlage mit einer erfindungsgemäßen Vorrichtung zum Anpassen der Reichweite eines Partikelstrahls an. Die Partikelstrahltherapieanlage ermöglicht damit im Rahmen einer Strahlentherapie eine schnelle und präzise Bestrahlung von bewegten Zielvolumina.

Die Erfindung beansprucht auch ein Verfahren zum Anpassen der Reichweite eines Partikelstrahls einer Partikelstrahltherapieanlage, indem die Reichweite eines Partikelstrahls durch mindestens zwei hintereinander angeordnete Reichweitenanpassmittel verändert wird.

In einer weiteren vorteilhaften Ausgestaltung der Erfindung können die Reichweitenanpassmittel derart moduliert werden, dass ein variabler Energieverlust einstellbar ist.

In einer besonderen Ausführungsform können unterschiedliche keilförmige Absorptionselemente der Reichweitenanpassmittel (11, 12) ineinander verfahren werden.

Weitere Besonderheiten und Vorteile der Erfindung werden aus den nachfolgenden Erläuterungen zu einem Ausführungsbeispiel anhand einer schematischen Zeichnung ersichtlich.

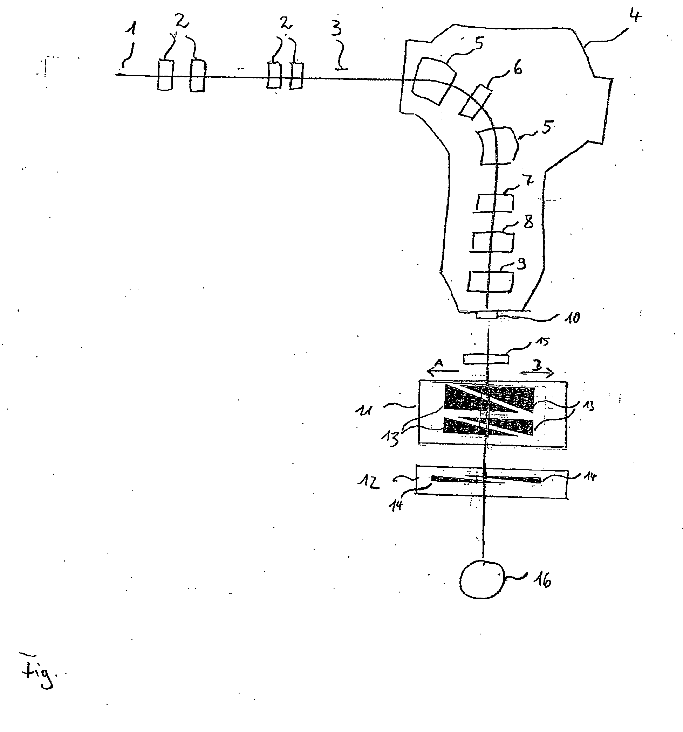

Die Zeichnung Figur zeigt eine Detailansicht einer beispielhaften Ausführungsform der erfindungsgemäßen Vorrichtung. Nach einer Erzeugung und Beschleunigung eines Partikelstrahls 1 wird sein Querschnitt durch Magnetsysteme 2 größenmäßig angepasst. Durch eine Schalteinheit 3 wird sichergestellt, dass der Partikelstrahl 1 jederzeit abgeschaltet werden kann. Anschließend gelangt der Partikelstrahl 1 in einen Strahlkopf 4, auch "Nozzle" genant. Der Strahlkopf 4 umfasst zwei Ablenkmagnete 5, einen den Partikelstrahl 1 fokussierenden Quadrupolmagneten 6, ein die Energie des Partikelstrahls 1 justierendes Einstellsystem 7, eine Kollimatoreinheit 8 zur Anpassung der Strahlform sowie ein Detektorsystem 9 zur Überwachung der abgegebenen Strahldosis. Anschließend verlässt der Partikelstrahl 1 den Strahlkopf 4 über ein Austrittsfenster 10. Durch ein erstes Reichweitenabsorptionselement in Form eines ersten keilförmigen Absorptionselements 11 und ein zweites Reichweitenabsorptionselement in Form eines keilförmiges Absorptionselements 12, die in dem Partikelstrahlgang zwischen dem Austrittsfenster 10 und dem Zielvolumen 16 angeordnet sind und quer zum Partikelstrahlzentrum zur Variation der Partikelstrahlenergie in Pfeilrichtung A und B verschiebbar sind, wird das Bragg-Maximum von der maximalen Eindringtiefe durch zunehmende Energieabsorption zu niedrigeren Eindringtiefen verschoben. Die Änderung der Energieabsorption wird dadurch erreicht, dass in den beiden keilförmigen Absorptionselementen 11 bzw. 12 jeweils paarweise gegenüberstehende Absorberkeile 13 bzw. 14 jeweils über eigene, nicht gezeigte Antriebsmotoren aufeinander zu bzw. voneinander weg bewegt werden, so dass ein in der Tiefe gestaffeltes Abtasten von Volumenelementen des Zielvolumens 16 in schneller Folge durchführbar wird. In einer Ionisationskammer 15 erfolgt die Messung der Anzahl der Partikel und deren Aufsummierung, bis eine vorbestimmte Partikelstrahldosis erreicht ist. Der so in seiner Reichweite angepasste Partikelstrahl trifft schließlich auf das Zielvolumen 16.

Um die Modulationsgeschwindigkeit zu maximieren und die Modulationspräzision zu optimieren, werden mehrere Reichweitenanpassmittel eingesetzt, deren kumulative Modulation die gewünschte Anpassung der Reichweite des Partikelstrahls ergibt. Die Reichweitenanpassmittel müssen nicht zwingend methodisch gleich oder gar identisch sein. Insbesondere kann es sinnvoll sein, die technischen Eigenschaften der Reichweitenanpassmittel gezielt zu variieren, um Modulationsgeschwindigkeit, Modulationspräzision, das Streuverhalten und/oder Größe des Überlappbereichs anzupassen. Werden z.B. Reichweitenanpassmittel mit jeweils mit opponierenden Absorberkeilen verwendet, so sollte vorzugsweise ein Reichweitenanpassmittel eine maximale Reichweitenmodulation von einigen Zentimetern (z.B. 4 cm) aufweisen, das andere Reichweitenanpassmittel hingegen nur eine geringere maximale Reichweitenmodulation (z.B. 1 cm). Dadurch verringert sich die Masse der Absorbermaterie des zweiten Reichweitenanpassmittels entscheidend, so dass eine schnellere Anpassung der Tiefenmodulation ermöglicht wird. Bei derart kombinierten Reichweitenanpassmitteln werden dann große Reichweitenanpassungen mit dem ersten, hingegen kleine Reichweitenanpassungen schneller mit dem zweiten Reichweitenanpassmittel durchgeführt.

Eine günstige Ausprägung kann dabei auch beinhalten, dass während des Trackings die beiden Systeme gegenläufig verfahren um zu verhindern, dass das dünnere Reichweitenanpassmittel den Maximalwert erreicht und die Bestrahlung unterbrochen werden muss. Das bedeutet, dass das dünne System kontinuierlich die Reichweite moduliert, gleichzeitig aber auch eine zweite Bewegungskomponente berücksichtigt, die umgekehrt zu der langsameren Bewegung des dickeren Reichweitenanpassmittels verläuft. Ziel dabei ist es, das dünnere Reichweitenanpassmittel immer um seine Mittellage der Reichweitenmodulation zu betreiben.

Des Weiteren kann es vorteilhaft sein, wenn mehrere Reichweitenanpassmittel so kombiniert werden, dass pro System Richtungswechsel vermieden werden, die zu den maximalen Anforderungen gehören (z.B. Bremsen, Stehen, Rückwärtsfahren der Linearmotoren, je mit voller Beschleunigung), aber in den Bestrahlungssitzungen regelmäßig vorkommen. Bei Kombination mehrerer Reichweitenanpassmittel können zeitintensive Positioniermanöver eines Reichweitenanpassmittels berücksichtigt und in Kauf genommen werden, wenn ein zweites Reichweitenanpassmittel den Spielraum hat, die geforderte Reichweitenänderung durchzuführen.

### Bezugszeichenliste

- 1: Partikelstrahl
- 2: Magnetsystem
- 3: Schalteinheit
- 4: Strahlkopf
- 5: Ablenkmagnet
- 6: Quadrupolmagnet
- 7: Einstellsystem
- 8: Kollimatoreinheit
- 9: Detektorsystem
- 10: Austrittsfenster
- 11: erstes keilförmiges Absorptionselement
- 12: zweites keilförmiges Absorptionselement
- 13: Absorberkeile des ersten keilförmigen Absorptionselements
- 14: Absorberkeile des zweiten keilförmigen Absorptionselements
- 15: Ionisationskammer
- 16: Zielvolumen

## Patentansprüche

1. Vorrichtung zum Anpassen der Reichweite eines Partikelstrahls (1) einer Partikelstrahltherapieanlage, **gekennzeichnet durch**:
mindestens zwei hintereinander angeordnete Reichweitenanpassmittel (11, 12), in denen der Partikelstrahl (1) beim Durchqueren einen Energieverlust erfährt.

2. Vorrichtung nach Anspruch 1, wobei die Reichweitenanpassmittel (11, 12) nach einem letzten Ablenkmagneten (5) angeordnet sind.

3. Vorrichtung nach Anspruch 1 **dadurch gekennzeichnet, dass** die Reichweitenanpassmittel (11, 12) derart modulierbar sind, dass der Energieverlust variabel einstellbar ist.

4. Vorrichtung nach einem der vorangegangenen Ansprüche, **dadurch gekennzeichnet, dass** die Reichweitenanpassmittel (11, 12) unterschiedliche keilförmige Absorptionselemente aufweisen, die ineinander verfahrbar sind.

5. Partikelstrahltherapieanlage mit einer Vorrichtung nach einem der vorhergehenden Ansprüche.

6. Verfahren zum Anpassen der Reichweite eines Partikelstrahls (1) einer Partikelstrahltherapieanlage, **dadurch gekennzeichnet, dass**
die Reichweite eines Partikelstrahls durch mindestens zwei hintereinander angeordnete Reichweitenanpassmittel (11, 12) verändert wird.

7. Verfahren nach Anspruch 6 **dadurch gekennzeichnet, dass** die Reichweitenanpassmittel (11, 12) derart moduliert werden, dass ein variabler Energieverlust einstellbar ist.

8. Verfahren nach Anspruch 6 oder 7 **dadurch gekennzeichnet, dass** unterschiedliche keilförmige Absorptionselemente der Reichweitenanpassmittel (11, 12) ineinander verfahren werden.
